(19) Europäisches Patentamt
European Patent Office
Office européen des brevets

(11) **EP 1 343 801 B1**

(12) **FASCICULE DE BREVET EUROPEEN**

(45) Date de publication et mention
de la délivrance du brevet:
**01.02.2012   Bulletin 2012/05**

(51) Int Cl.:
*C07H 21/00* (2006.01)          *C07B 61/00* (2006.01)
*C07F 7/18* (2006.01)          *G01N 33/53* (2006.01)
*C12Q 1/68* (2006.01)

(21) Numéro de dépôt: **01994934.6**

(22) Date de dépôt: **20.12.2001**

(86) Numéro de dépôt international:
**PCT/FR2001/004112**

(87) Numéro de publication internationale:
**WO 2002/051856 (04.07.2002 Gazette 2002/27)**

(54) **PROCEDE D'IMMOBILISATION DE SONDES, EN PARTICULIER POUR REALISER DES PUCES BIOLOGIQUES**

VERFAHREN ZUR IMMOBILISIERUNG VON SONDEN, INSBESONDERE ZUM SCHAFFEN VON BIOLOGISCHEN CHIPS

METHOD FOR IMMOBILISING PROBES, IN PARTICULAR FOR PRODUCING BIOCHIPS

(84) Etats contractants désignés:
**AT BE CH CY DE DK ES FI FR GB GR IE IT LI LU MC NL PT SE TR**

(30) Priorité: **22.12.2000   FR 0016940**

(43) Date de publication de la demande:
**17.09.2003   Bulletin 2003/38**

(73) Titulaire: **Commissariat à l'Énergie Atomique et aux Énergies Alternatives**
**75015 Paris (FR)**

(72) Inventeurs:
• **VINET, Françoise**
**F-38000 GRENOBLE (FR)**
• **HOANG, Antoine**
**F-38000 GRENOBLE (FR)**

(74) Mandataire: **Poulin, Gérard et al**
**BREVALEX**
**95, rue d'Amsterdam**
**75378 Paris Cedex 8 (FR)**

(56) Documents cités:
**EP-A- 0 127 438          WO-A-81/01860**
**WO-A-99/37630          US-A- 5 958 792**

• **MASKOS U ET AL: "OLIGNUCLEOTIDE HYBRIDISATIONS ON GLASS SUPPORTS: A NOVEL LINKER FOR OLIGONUCLEOTIDE SYNTHESIS AND HYBRIDISATION PROPERTIES OF OLIGNUCLEOTIDES SYNTHESISED IN SITU" NUCLEIC ACIDS RESEARCH, OXFORD UNIVERSITY PRESS, SURREY, GB, vol. 20, no. 7, 1992, pages 1679-1684, XP000651031 ISSN: 0305-1048**
• **REDEMANN, THOMAS ET AL: "Synthesis of peptidomimetics using a polymer-bound Boc-linker" MOL. DIVERSITY (2000), VOLUME DATE 1998, 4(3), 191-197, XP001015985**
• **REGGELIN M ET AL: "Towards Polyketide Libraries-II: Synthesis of Chiral Aracemic Di- and Triketides on a Solid Support" TETRAHEDRON LETTERS, ELSEVIER SCIENCE PUBLISHERS, AMSTERDAM, NL, vol. 39, no. 27, 2 juillet 1998 (1998-07-02), pages 4801-4804, XP004120761 ISSN: 0040-4039**
• **UKEDA, HIROYUKI ET AL: "Direct immobilization of NAD to Sepharose 4B by using the bifunctional reagent glutaraldehyde" AGRIC. BIOL. CHEM. (1989), 53(1), 235-7, XP002180871**
• **SMITH, AMOS B.,III ET AL: "Synthesis of Polypyrrolinones on Solid Support" ORG. LETT. (2000), 2(14), 2041-2044, XP002180872**

## Description

**DOMAINE TECHNIQUE**

L'invention concerne un procédé d'immobilisation de sondes en particulier pour réaliser des puces biologiques. Elle permet la fabrication de biopuces à oligonucléotides dont le greffage sur un support solide est réalisé par l'intermédiaire d'une liaison covalente.

**ETAT DE LA TECHNIQUE ANTERIEURE**

Les biopuces peuvent être réalisées par synthèse parallèle d'oligonucléotides directement sur un support solide. Dans ce cas, il existe une limitation du procédé liée à la pureté des sondes synthétisées. En effet, aucune purification après synthèse n'est possible. Ceci engendre la présence sur le support solide de séquences tronquées de longueur (n-1) mères pour une longueur visée de n. Cette technique est adaptée à des puces de haute densité (supérieure à 1000 sondes), dont les sondes ont une longueur comprise entre 6 et 20 mères. Les applications visées dans ce cas, sont le séquençage et le criblage (ou "screening" en anglais) du polymorphisme simple de nucléotide (SNP).

Une autre voie est l'immobilisation de sondes. Dans ce cas, les sondes sont présynthétisées et peuvent donc être purifiées. Deux types d'immobilisation de sondes oligonucléotides se présentent. Une sonde purifiée peut être fixée sur un support solide soit par adsorption, soit par greffage via une réaction spécifique entre la sonde et le support.

L'adsorption de la sonde sur le support est un phénomène passif puisque des liaisons de type électrostatique établies entre le squelette phosphodiester de la sonde chargée négativement et le support modifié portant des charges positives interviennent de façon non covalente dans l'immobilisation de la sonde. Ce type d'immobilisation est compatible avec des longueurs de sondes comprises entre 70 et quelques centaines de mères.

Par contre, dans le cas d'un greffage de la sonde par réaction spécifique, le phénomène est dit actif, car il est dû à la réactivité et à la spécificité entre la fonction branchée sur la sonde et celle introduite sur le support solide. Ce mode d'immobilisation établit par conséquent des liaisons de type covalent entre la sonde déposée et le support utilisé. Il est compatible avec des longueurs de sondes comprises entre 6 et 50 à 60 mères.

L'immobilisation de sondes oligonucléotidiques met en oeuvre un couple de fonctions chimiques branchées sur la sonde et le support. Cette notion de couple de fonctions chimiques s'appuie sur la réactivité entre un nucléophile et un électrophile.

Il existe donc plusieurs possibilités de fonctionnalisation, c'est-à-dire d'apport des fonctions chimiques sur la sonde et sur le support. On peut ainsi utiliser une sonde nucléophile sur un support électrophile, une sonde électrophile sur un support nucléophile, une sonde nucléophile sur un support également nucléophile mais en présence d'un bras di-électrophile, et une sonde électrophile sur un support également électrophile mais en présence d'un bras di-nucléophile.

Les sondes rendues nucléophiles par une fonction amine sont les plus répandues. En effet, cette fonction est stable et est très accessible commercialement.

Différentes étapes sont nécessaires à l'obtention des plots fluorescents correspondant aux hybridons, produits issus de l'appariement entre deux simples brins d'ADN complémentaires.

L'obtention d'un support fonctionnalisé pour biopuce à immobilisation de sondes nécessite 3 étapes principales :

- le nettoyage de la surface du support devant présenter les sites réactifs,
- la silanisation de cette surface,
- l'activation des sites.

Ces étapes sont suivies de 3 autres étapes qui vont conduire à l'acquisition des signaux de fluorescence :

- l'immobilisation des sondes sur les sites,
- l'hybridation des sondes,
- la lecture.

On distingue principalement deux voies d'introduction de sites d'ancrage des sondes sur le support à partir desquels des fonctions chimiques seront créées ultérieurement. Ces sites sont soit nucléophiles, soit électrophiles. Ils sont apportés lors de l'étape de silanisation.

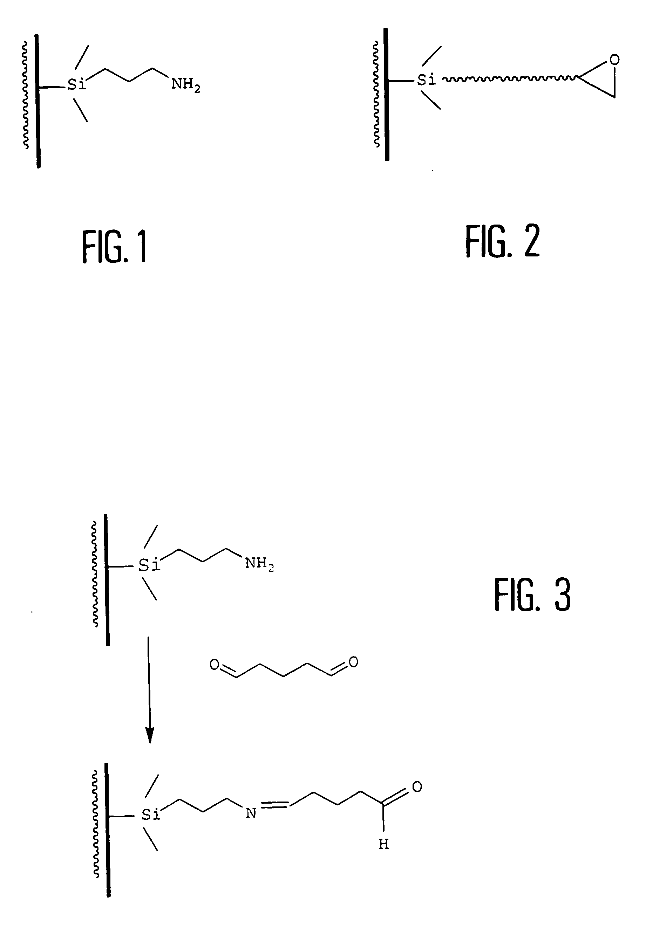

A titre d'exemple, la figure 1 représente un site nucléophile constitué par une fonction amine rattachée à un support par un silane.

Des supports pour biopuces actuellement disponibles présentent des sites électrophiles par apport d'une fonction aldéhyde. Ils permettent l'accrochage des sondes rendues nucléophiles par une fonction amine.

La figure 3 représente un schéma d'obtention d'une fonction aldéhyde sur un support solide à partir d'une fonction amine couplée avec un bras dialdéhyde.

Les supports pour biopuces offrant une fonction aldéhyde et actuellement connus présentent les inconvénients suivants.

Ils nécessitent un intermédiaire de couplage (voir la figure 3), ce qui diminue la densité finale de sites d'accrochage des sondes.

Leur stockage pose problème, la forme aldéhyde étant relativement peu stable. En effet, il a été observé que les supports doivent être utilisés immédiatement après l'ouverture de la boîte qui les contient.

Le groupement $NH_2$ initialement sur le support (voir la figure 3) peut être transformé en $NH_3^+$, ce qui favorise l'adsorption de composés non spécifiques. Il en résulte une augmentation du bruit de fond des biopuces.

EP 0 127 438 AI décrit la modification d'un support à base de silice par un traitement au gamma-glycidoxypropyl monométhoxysilane. Une hydrolyse de l'époxyde en milieu acide conduit à un diol séparé du support solide par un groupement - $(CH_2)_3OCH_2$- qui comporte un atome d'oxygène.

## EXPOSÉ DE L'INVENTION

Afin d'apporter une solution aux inconvénients présentés par l'art connu, il est proposé selon la présente invention d'obtenir une fonction aldéhyde (permettant l'immobilisation d'une sonde) par transformation d'un époxyde en passant par un diol.

Un premier objet de l'invention consiste en un support solide constitué par un substrat comportant des sites destinés à l'immobilisation de sondes oligonucléotidiques ou de protéines ou de cellules biologiques munies de ligands, les sites étant pourvus d'espèces chimiques accrochées au substrat par une fonction chimique d'accrochage, caractérisé en ce que les espèces chimiques présentent un groupe diol lié au support par une chaîne alkyle.

La figure 2 représente un site électrophile constitué par une fonction époxyde rattachée à un support par un groupe silane disposé au bout d'une chaîne alkyle. La chaîne alkyle est exempte d'oxygène.

Un tel support présente l'avantage de pouvoir être stocké sans problème pendant six mois, la forme diol étant plus stable que la forme aldéhyde.

Le substrat peut être en un matériau choisi parmi le verre, le silicium et le pastique.

De préférence, la fonction chimique d'accrochage des espèces chimiques sur le support est une fonction silane.

Un deuxième objet de l'invention consiste en un support solide constitué par un substrat comportant des sites activés pour l'immobilisation de sondes oligonucléotidiques ou de protéines ou de cellules biologiques munies de ligands, les sites étant pourvus d'espèces chimiques accrochées au substrat par une fonction chimique d'accrochage, les espèces chimiques comportant un groupe aldéhyde destiné à l'immobilisation desdites sondes, caractérisé en ce que le groupe aldéhyde est un groupe obtenu par oxydation d'un diol porté par une chaîne alkyle. La chaîne alkyle évite, avant l'étape d'oxydation, la présence d'oxygène sur le lien entre la fonction diol et le groupe d'accrochage sur le support.

Le substrat peut être en un matériau choisi parmi le verre, le silicium et le plastique revêtu ou non d'une couche d'accrochage.

De préférence, la fonction chimique d'accrochage des espèces chimiques sur le support est une fonction silane.

Un troisième objet de l'invention consiste en une biopuce comprenant un support solide constitué par un substrat comportant des sites activés comme défini ci-dessus, des sondes oligonucléotidiques ou des protéines ou des cellules biologiques munies de ligands étant immobilisées sur les sites activés grâce à une liaison covalente entre les fonctions aldéhyde du support solide et des fonctions amine portées par les sondes.

Un quatrième objet de l'invention consiste en un procédé de fabrication d'un support solide selon la revendication 1 , comprenant les étapes suivantes :

- nettoyage d'une surface d'un substrat,
- définition desdits sites par mise en place, sur la surface nettoyée, d'espèces chimiques accrochées au substrat par une fonction chimique d'accrochage, les espèces chimiques comportant un précurseur destiné à former une fonction aldéhyde permettant l'immobilisation des sondes, des protéines ou des cellules biologiques munies de ligands, caractérisé en ce que le précurseur est une fonction époxyde et est traité pour donner un groupe diol, le précurseur époxyde étant disposé au bout d'une chaîne alkyle.

Avantageusement, le traitement pour donner un groupe diol est une hydrolyse acide de l'époxyde.

L'étape de nettoyage peut consister à nettoyer une surface d'un substrat en un matériau choisi parmi le verre, le silicium et le plastique.

De préférence, la fonction chimique d'accrochage des espèces chimiques sur le substrat est une fonction silane.

Le procédé peut comporter en outre une étape consistant à oxyder le diol pour obtenir un aldéhyde.

Un cinquième objet de l'invention consiste en un procédé de fabrication d'une biopuce selon la revendication 7, comprenant :

- la fabrication d'un support solide présentant des sites destinés à l'immobilisation de sondes oligonucléotidiques ou de protéines ou de cellules biologiques munies de ligands selon le procédé ci-dessus,
- l'immobilisation des sondes oligonucléotidiques ou des protéines ou des cellules biologiques munies de ligands par liaison covalente entre les fonctions aldéhyde du support solide et des fonctions amine (ou oxyamine) portées par les sondes en position 5' ou 3'.

## BRÈVE DESCRIPTION DES DESSINS

L'invention sera mieux comprise et d'autres avantages et particularités apparaîtront à la lecture de la description qui va suivre, donnée à titre d'exemple non limitatif, accompagnée des dessins annexés parmi lesquels :

- la figure 1, déjà décrite, représente un site nucléophile constitué par une fonction amine rattachée à un support par un silane,

- la figure 2, déjà décrite, représente un site électrophile constitué par une fonction époxyde rattachée à un support par un silane,
- la figure 3, déjà décrite, représente un schéma d'obtention d'une fonction aldéhyde sur un support solide, selon l'art connu,
- la figure 4 représente un schéma d'obtention d'une fonction aldéhyde sur un support solide, selon l'invention,
- la figure 5 est un schéma montrant la fonction diol en position vicinale,
- la figure 6 est un schéma montrant le mécanisme de complexation du periodate de sodium sur un diol,
- la figure 7 est un schéma montrant la fonctionnalisation d'un substrat silanisé par le procédé de l'invention.

## DESCRIPTION DETAILLEE DE MODES DE REALISATION DE L'INVENTION

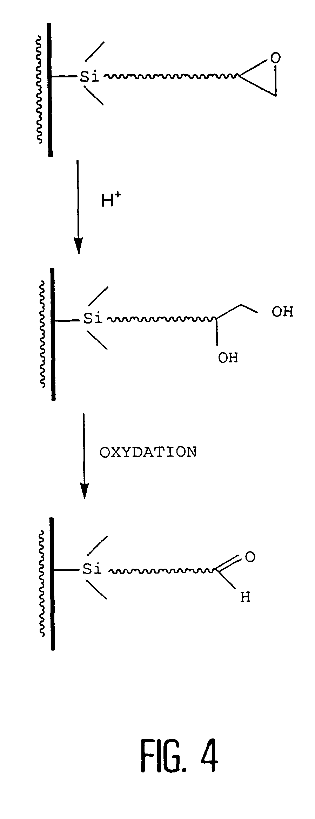

La figure 4 est un schéma d'obtention d'une fonction aldéhyde sur un support solide à partir d'une fonction époxyde hydrolysée en diol puis oxydé en aldéhyde, selon l'invention. L'époxyde utilisé est par exemple du glycidoxypropylépoxyde triétoxysilane.

Cependant, les inventeurs de la présente invention ont constaté qu'après les étapes de nettoyage, de silanisation, d'activation puis d'hybridation, les résultats de fluorescence des plots d'hybridation sur le support sont peu encourageants. Ceci n'est pas dû à l'étape d'hybridation puisque des manipulations parallèles effectuées sur des lames commerciales et des lames aminées, ont donné de meilleures intensités de signaux.

Les inventeurs ont donc remis en cause, non pas les étapes de nettoyage ou de silanisation (qui sont efficaces en synthèse in situ), mais plutôt l'étape d'activation, c'est-à-dire lors de la transformation de la fonction époxyde en aldéhyde et plus précisément, l'oxydation du diol vicinal du silane en aldéhyde.

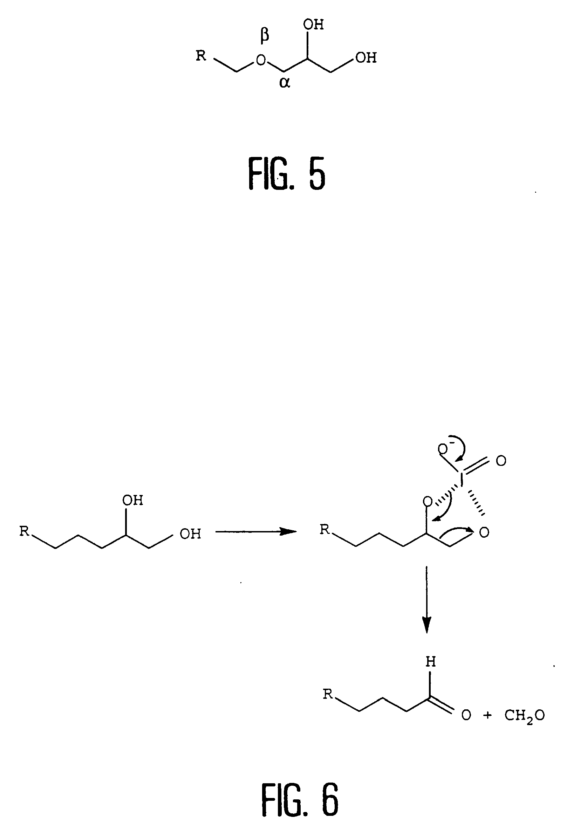

La figure 5 est un schéma montrant la fonction diol en position vicinale.

Il en résulte qu'avec un époxyde sans oxygène sur la chaîne alkyle qui porte le groupe, l'oxydation doit avoir lieu.

La figure 6 est un schéma montrant le mécanisme de complexation du periodate de sodium sur un diol.

Pour vérifier leur hypothèse, les inventeurs ont utilisé un silane portant une fonction époxyde liée par une chaîne alkyle (en l'absence donc d'oxygène sur cette chaîne).

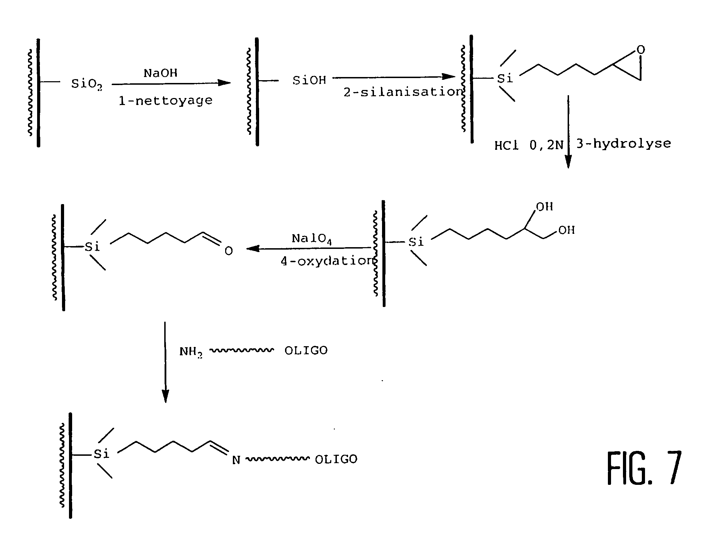

La figure 7 est un schéma montrant la fonctionnalisation d'un substrat silanisé selon l'invention.

Après les différentes étapes de la chaîne réactionnelle, on a obtenu des plots de fluorescence saturés sur fond noir, ce qui confirme l'hypothèse des inventeurs.

L'invention présente plusieurs avantages par rapport à l'état de la technique et plus particulièrement par rapport aux substrats de type aldéhyde actuellement commercialisés.

L'invention n'utilise pas d'intermédiaire de couplage qui diminue la densité finale de sites d'accrochage des sondes. Le signal de fluorescence obtenu est plus important dans le cas de l'invention. On observe un facteur 10 dans des conditions expérimentales identiques.

Comme mentionné plus haut, l'invention permet de stocker les supports solides sous la forme diol qui est particulièrement stable.

L'invention permet aussi de minimiser le bruit de fond des biopuces puisqu'il n'y a pas de groupement permettant des interactions électrostatiques.

La préparation des supports selon l'invention est transposable à d'autres techniques de réalisation de biopuces par exemple la synthèse in situ.

A titre d'exemple, après ouverture de l'époxyde en diol, le silane selon l'invention a été utilisé pour réaliser la synthèse in situ d'oligonucléotides sur un support solide. On a mis en oeuvre la chimie phosphoramidite couramment utilisée pour la synthèse des oligonucléotides sur billes de verre dans des synthétiseurs automatiques du type Expedite 8909. Le protocole de synthèse utilisé est décrit dans le document Caruthers (Sciences, octobre 1985, page 28) 1. Il comporte les étapes de couplage, d'acétylation, d'oxydation et de détritylation, ces étapes étant itérées pour permettre la synthèse d'oligonuléotides dont la séquence est déterminée par la base (A, T, G ou C) portée par le nucléotide phosphoramidite.

La première étape de couplage se fait entre un 2'-déoxy-5'-O-diméthoxytrityl-3'-O-(β-cyanoéthyl N,N-diisopropylamino) phosphoramidite et le diol du support. Les couplages suivants ont lieu entre les nucléotides comportant des bases différentes. Une séquence de 20 mères a été effectuée : 3'TTTTT ATC TCA CAC AAA TAG Cy3 5'. En fin de synthèse un marqueur fluorescent Cy3 a été introduit en utilisant comme précédemment la chimie phosphoramidite.

Les signaux de fluorescence mesurés sur un microscope à épifluorescence Olympus BX80 après excitation à 550 nm montrent une augmentation d'un facteur 4 à 5 fois supérieur à ceux obtenus dans le cas d'un silane comportant un oxygène sur la chaîne alkyle.

## Revendications

1. Support solide constitué par un substrat comportant des sites destinés à l'immobilisation de sondes oligonucléotidiques ou de protéines ou de cellules biologiques munies de ligands, les sites étant pourvus d'espèces chimiques accrochées au substrat par une fonction chimique d'accrochage, **caractérisé en ce que** les espèces chimiques présentent un groupe diol lié au support par une chaîne alkyle.

2. Support solide selon la revendication 1, **caractérisé**

**en ce que** le substrat est en un matériau choisi parmi le verre, le silicium et le pastique.

3. Support solide selon l'une des revendications 1 ou 2, **caractérisé en ce que** ladite fonction chimique d'accrochage des espèces chimiques sur le support est une fonction silane.

4. Support solide constitué par un substrat comportant des sites activés pour l'immobilisation de sondes oligonucléotidiques ou de protéines ou de cellules biologiques munies de ligands, les sites étant pourvus d'espèces chimiques accrochées au substrat par une fonction chimique d'accrochage, les espèces chimiques comportant un groupe aldéhyde destiné à l'immobilisation desdites sondes, **caractérisé en ce que** le groupe aldéhyde est un groupe obtenu par oxydation d'un diol porté par une chaîne alkyle.

5. Support solide selon la revendication 4, **caractérisé en ce que** le substrat est en un matériau choisi parmi le verre, le silicium et le plastique revêtu ou non d'une couche d'accrochage.

6. Support solide selon l'une des revendications 4 ou 5, **caractérisé en ce que** ladite fonction chimique d'accrochage des espèces chimiques sur le support est une fonction silane.

7. Biopuce, **caractérisée en ce qu'**elle comprend un support solide selon l'une quelconque des revendications 4 à 6, des sondes oligonucléotidiques ou des protéines ou des cellules biologiques munies de ligands étant immobilisées sur les sites activés grâce à une liaison covalente entre les fonctions aldéhyde du support solide et des fonctions amine portées par les sondes.

8. Procédé de fabrication d'un support solide selon la revendication 1 , comprenant les étapes suivantes :

   - nettoyage d'une surface d'un substrat,
   - définition desdits sites par mise en place, sur la surface nettoyée, d'espèces chimiques accrochées au substrat par une fonction chimique d'accrochage, les espèces chimiques comportant un précurseur destiné à former une fonction aldéhyde permettant l'immobilisation des sondes, des protéines ou des cellules biologiques munies de ligands, **caractérisé en ce que** le précurseur est une fonction époxyde et est traité pour donner un groupe diol, le précurseur époxyde étant disposé au bout d'une chaîne alkyle.

9. Procédé selon la revendication 8, **caractérisé en ce que** le traitement pour donner un groupe diol est une hydrolyse de l'époxyde.

10. Procédé selon l'une des revendications 8 ou 9, **caractérisé en ce que** l'étape de nettoyage consiste à nettoyer une surface d'un substrat en un matériau choisi parmi le verre, le silicium et le plastique.

11. Procédé selon l'une quelconque des revendications 8 à 10, **caractérisé en ce que** la fonction chimique d'accrochage des espèces chimiques sur le substrat est une fonction silane.

12. Procédé selon l'une quelconque des revendications 8 à 11, **caractérisé en ce qu'**il comporte en outre une étape consistant à oxyder le diol vicinal pour obtenir un aldéhyde.

13. Procédé de fabrication d'une biopuce selon la revendication 7, **caractérisé en ce qu'**il consiste à :

   - fabriquer un support solide présentant des sites destinés à l'immobilisation de sondes oligonucléotidiques ou de protéines ou de cellules biologiques munies de ligands selon le procédé de la revendication 12,
   - immobiliser des sondes oligonucléotidiques, des protéines ou des cellules biologiques munies de ligands par liaison covalente entre les fonctions aldéhyde du support solide et des fonctions amine portées par les sondes, les protéines ou les cellules biologiques munies de ligands en position 5' ou 3'.

**Claims**

1. Solid support constituted by a substrate comprising sites intended for immobilization of oligonucleotide probes, proteins, or biological cells having ligands, the sites being provided with chemical species bound to the substrate by a chemical binding function, **characterized in that** the chemical species have a diol group connected to the support by an alkyl chain.

2. Solid support according to claim 1, **characterized in that** the substrate is a material chosen from among glass, silicon and plastic.

3. Solid support according to one of claims 1 or 2, **characterized in that** said chemical function for binding the chemical species to the support is a silane function.

4. Solid support constituted by a substrate comprising sites activated for the immobilization of oligonucleotide probes, proteins, or biological cells having ligands, the sites being provided with chemical species bound to the substrate by a chemical binding function, the chemical species comprising an aldehyde group intended for immobilization of said probes, **characterized in that** the aldehyde group is a group obtained by oxidation of a diol carried by an alkyl chain.

5. Solid support according to claim 4, **characterized in that** the substrate is a material chosen from among glass, silicon and plastic, coated or not with a binding layer.

6. Solid support according to one of claims 4 or 5, **characterized in that** said chemical function for binding chemical species to the support is a silane function.

7. Biochip, **characterized in that** it comprises a solid support according to any one of claims 4-6, oligonucleotide probes, proteins, or biological cells having ligands being immobilized on the activated sites by means of a covalent bond between the aldehyde functions of the solid support and amine functions carried by the probes.

8. Method of manufacture of a solid support according to claim 1, comprising the following steps:

- cleaning a surface of a substrate,
- defining said sites by positioning on the cleaned surface chemical species bound to the substrate by a chemical binding function, the chemical species comprising a precursor intended to forum an aldehyde function permitting the immobilization of probes, proteins, or biological cells having ligands, **characterized in that** the precursor is an epoxide function and is treated to give a diol group, the epoxide precursor being disposed at the end of an alkyl chain.

9. Method according to claim 9, **characterized in that** the treatment to give a diol group is a hydrolysis of the epoxide.

10. Method according to one of claims 8 or 9, **characterized in that** the cleaning step consists of cleaning a surface of a substrate of a material chosen from among glass, silicon, and plastic.

11. Method according to any one of claims 8-10, **characterized in that** the chemical function for binding of the chemical species to the substrate is a silane function.

12. Method according to any one of claims 8-11, **characterized in that** it further comprises a step consisting of oxidizing the vicinal diol to obtain an aldehyde.

13. Method of manufacture of a biochip according to claim 7, **characterized in that** it consists of:

- manufacturing a solid support having sites intended for the immobilisation of oligonucleotide probes, proteins, or biological cells having ligands, according to the method of claim 12,
- immobilizing oligonucleotide probes, proteins, or biological cells having ligands by covalent bond between the aldehyde functions of the solid support and amine functions carried by the probes, proteins, or biological cells having ligands in the 5' or 3' position.

**Patentansprüche**

1. Fester Träger bestehend aus einem Substrat, welches Sites bzw. Bindungsstellen aufweist, die zur Immobilisation von Oligonucleoditischen Sonden oder von Proteinen oder von mit Liganden versehenen biologischen Zellen bestimmt sind, wobei die Bindungssites bzw. -stellen mit chemischen Spezies versehen sind, die durch eine chemische Kopplungsfunktion mit dem Substrat gekoppelt sind, **dadurch gekennzeichnet dass** die chemischen Spezies eine Diol-Gruppe aufweisen, die durch eine Alkyl-Kette an den Träger gebunden ist.

2. Fester Träger nach Anspruch 1 , **dadurch gekennzeichnet, dass** das Substrat aus einem Material besteht, das aus Glas, aus Silizium und aus Kunststoff gewählt ist.

3. Fester Träger nach einem der Ansprüche 1 oder 2, **dadurch gekennzeichnet, dass** die genannte chemische Funktion zur Kopplung der chemischen Spezies an dem Träger eine Silan-Funktion ist.

4. Fester Träger bestehend aus einem Substrat, welches Sites bzw. Bindungsstetlen aufweist, die zur Immobilisation von Oligonucleoditischen Sonden oder von Proteinen oder von mit Liganden versehenen biologischen Zellen aktiviert sind, wobei die Bindungssites bzw. -stellen mit chemischen Spezies versehen sind, die durch eine chemische Kopplungsfunktion mit dem Substrat gekoppelt sind, und wobei die chemischen Spezies eine zur Immobilisierung der genannten Sonden bestimmte Aldehydgruppe aufweisen, **dadurch gekennzeichnet, dass** die Aldehydgruppe eine durch Oxydation eines von einer Alkylkette getragenen Diols erhaltene Gruppe ist.

5. Fester Träger nach Anspruch 4 , **dadurch gekennzeichnet, dass** das Substrat aus einem Material besteht, das aus Glas, Silizium und einem Kunststoff ausgewählt ist und gegebenenfalls mit einer Kopplungsschicht überzogen ist.

6. Fester Träger nach einem der Ansprüche 4 oder 5, **dadurch gekennzeichnet, dass** die genannte chemische Funktion für die Kopplung die chemischen Spezies auf dem Träger eine Silanfunktion ist.

7. Bio-Chip, **dadurch gekennzeichnet, dass** er einen festen Träger nach einem beliebigen der Ansprüche 4 bis 6 umfasst, wobei Oligonukleotid-Sonden oder Proteine oder mit Liganden versehenen biologischen Zellen an den aktiven Sites bzw. Stellen auf Grund einer kovalenten Bindung zwischen den Aldehyd-Funktionen des festen Trägers und den von den Sonden getragenen Amin-Funktionen immobilisiert sind.

8. Verfahren zur Herstellung eines festen Trägers nach Anspruch 1, welches die folgenden Stufen bzw. Schritte umfasst:

   - - Reinigen einer Oberfläche eines Substrats,
   - - Definition der genannten Sites bzw. Anbindungsstellen , indem auf der gereinigten Oberfläche chemische Spezies angebracht werden, die durch eine chemische Kopplungsfunktion an das Substrat gekoppelt sind, wobei die chemischen Spezies einen Vorläufer umfassen, der zur Bildung einer Aldehydfunktion bestimmt ist, welche die Immobilisierung der Sonden, der Proteine oder der mit Liganden versehenen biologischen Zellen gestatten, **dadurch gekennzeichnet, dass** der Vorläufer eine Epoxydfunktion ist und zur Bindung einer Diolgruppe behandelt wird, wobei der Epoxydvorläufer am Ende einer Alkylkette angeordnet ist.

9. Verfahren nach Anspruch 8, **dadurch gekennzeichnet, dass** die Behandlung zur Bildung einer Diolgruppe eine Hydrolyse des Epoxyds ist.

10. Verfahren nach einem der Ansprüche 8 oder 9, **dadurch gekennzeichnet, dass** die Reinigungsstufe in der Reinigung einer Oberfläche eines Substrats aus einem aus Glas, Silizium und Kunststoff gewählten Material besteht.

11. Verfahren nach einem beliebigen der Ansprüche 8 bis 10, **dadurch gekennzeichnet, dass** die chemische Funktion für die Kopplung der chemischen Spezies an dem Substart eine Silanfunktion ist.

12. Verfahren nach einem beliebigen der Ansprüche 8 bis 11, **dadurch gekennzeichnet, dass** es des weiteren eine Verfahrensstufe umfasst, welche in der Oxydation des benachbarten Diols besteht, um einen Aldehyd zu erhalten.

13. Verfahren zur Herstellung eines Bio-Chips nach Anspruch 7, **dadurch gekennzeichnet, dass** es darin besteht:

- - Herstellen eines festen Trägers, welcher Sites bzw. Bindungsstellen aufweist, die zur Immobilisierung von Oligonukleotid-Sonden, oder von Proteinen oder von mit Liganden versehenen biologischen Zellen bestimmt sind, gemäß dem Verfahren nach Anspruch 12 ,
- - Immobilisieren der Oligonukleotidsonden, der Proteine oder der mit Liganden versehenen biologischen Zellen durch kovalente Bindung zwischen den Aldehydfunktionen des festen Trägers und den Aminfunktionen, die von den Sonden, den Proteinen oder den mit Liganden versehenen biologischen Zellen getragen sind, in 5'- oder 3'-Stellung.

FIG. 1

FIG. 2

FIG. 3

FIG. 4

FIG. 5

FIG. 6

FIG. 7

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 0127438 A1

**Littérature non-brevet citée dans la description**

- **CARUTHERS.** *Sciences,* Octobre 1985, 28